# EUROPEAN PATENT APPLICATION

(11) **EP 1 197 554 A1**
(43) Date of publication of application: **17.04.2002**
(21) Application number: 00944306.0
(22) Date of filing: 06.07.2000
(51) Int. Cl.: C12N 15/16

(54) **PROLIFERATION DIFFERENTIATION FACTOR**

(30) Priority: 08.07.1999 JP 19417999; 18.10.1999 US 159586 P
(71) Applicant: Helix Research Institute, Kisarazu-shi, Chiba 292-0812 (JP)
(72) Inventor: OTA, Toshio, Kyowa Hakko Kogyo Co,Ltd, Machida-shi, Tokyo 194-8533 (JP); ISOGAI, Takao, Inashiki-gun, Ibaraki 300-0303 (JP); NISHIKAWA, Tetsuo, Itabashi-ku, Tokyo 173-0013 (JP); HIO,Yuri, Kisarazu-shi, Chiba 292-0812 (JP); YOSHIDA, Kenji, Inashiki-gun, Ibaraki 300-1241 (JP); MASUHO, Yasuhiko, Koganei-shi, Tokyo 184-0011 (JP)
(74) Representative: VOSSIUS & PARTNER
(86) International application number: PCT/JP00/04514
(87) International publication number: WO 01/04312

(57) **Abstract**

A protein encoded by PSEC137 that is cloned from a full-length human cDNA library. The protein is a novel protein having a thorombopoietin (TPO)/ erythropopoietin (EPO)-like amino acid sequence. The present protein is expected as a novel hematopoietic factor inducing the differentiation of blood precursor cells, etc.

## Description

### Technical Field

The present invention relates to genes that encode growth and differentiation factors.

### Background Art

The formation of blood cells comprises a process in which few hematopoietic stem cells produce progenitor cells of a particular cell line, and following growth and differentiation of these cells, mature blood cells are produced. This process is regulated by the function of multiple hormones that act specifically, and the general term for these hormones is "growth and differentiation factors" or "colony-stimulating factors" (Dexter (1989) Br. Med. Bull. 45, 337; Ogawa (1989) Environ. Health Presp. 80, 199; Metcalf (1985) Science 229, 16; Golde and Gasson (1988) Scientific American July, 62).

Growth and differentiation factors are humoral factors that transmit growth and differentiation signals to various cells. For example, erythropoietin (EPO) was isolated as a factor that promotes growth and differentiation of erythroid progenitor cells. EPO, which, later, has been utilized as a hematopoietic factor in the treatment of anemia, is an important growth and differentiation factor.

The existence of a factor that promotes growth of megakaryocytic cells was predicted, while EPO acts upon erythroid progenitor cells. Subsequently, a protein encoded by a gene that was isolated as a c-mpl ligand was found to have a proliferative effect on megakaryocytic cells. The c-mpl ligand was elucidated to be a megakaryocytic growth factor, and was identified as thrombopoietin (TPO)(Lok et al. (1994) Nature 369, 568; Bartley et al. (1994) Cell 77, 1117; de Sauvage et al. (1994) Nature 369, 533). Megakaryocytic cells are cells that are involved in thrombocytopoiesis, and such. It is expected that TPO enable to treatthrombocytopenia and such, caused by side effects of anticancer drug administration, may become possible.

The N-terminal region (amino acid residues 1-172) of human TPO shows 23% sequence homology to human EPO (Gurney et al. (1995) Blood 85, 981-988; Bartley et al. (1994) Cell 77, 1117-1124; de Sauvage et al. (1994) Nature 369, 533), and forms a family within the group in growth and differentiation factors. However, since then, few reports for growth and differentiation factors that belong to this type of EPO/ TPO family have been provided. Novel growth and differentiation factors may be different from known factors in terms of the strength of growth and differentiation inducing activity thereof, the spectrum of cells upon which they act, etc. Therefore, isolation of novel factors has been desired.

### Disclosure of the Invention

The object of the present invention is to provide a growth and differentiation factor and a gene encoding it, as well as method for producing and utility thereof. Novel growth and differentiation factors or compounds that modify their activity or expression are expected as therapeutic agents for diseases that accompany abnormalities of blood cells.

Therefore, the present inventors made the effort to carry out the following research that aims to clone novel human genes in order to solve the objectivesmentioned above. First, the inventors isolated a clone comprising a full-length-enriched cDNA library that is synthesized by the oligo-capping method (Maruyama, K. and Sugano, S., Gene 138: 171-174, 1994; Suzuki, Y. et al., Gene 200: 149-156, 1997). Then, the inventors determined the nucleotide sequence of the obtained full-length-enriched cDNA clones from both 5' and 3' ends. Then, human full-length DNA, expected to be a full-length DNA by using ATGpr (Salamov, A. A. et al. Bioinfomatics 14: 384-390, 1998; http://www.hri.co.jp/atgpr/) and so forth, was selected. By utilizingthe resulting sequences of full-length-enriched cDNA clones, the inventors selected clones that were expected to contain a signal by the PSORT (Nakai, K. and Kanehisa, M. Genomics 14: 897-911 1992), and obtained clones that contain a cDNA encoding a secretory protein. The inventors have analyzed the nucleotide sequence of the full-length cDNA clones, and deduced the amino acid sequence encoded by the nucleotide sequence. Then, the inventors have performed the BLAST search (Altschul, S. F., et al. J. Mol. Biol. 215: 403-410, 1990; Gish, W., and States, D.J. Nature Genet. 3: 266-272 1993; http://www.ncbi.nlm.nih.gov/BLAST/) of the GenBank (http://www.ncbi.nlm.nih.gov/Web/GenBank/index.html) and SwissProt (http://www.ebi.ac.uk/ebi_docs/swissprot_db/swisshome.html) using the deduced amino acid sequence.

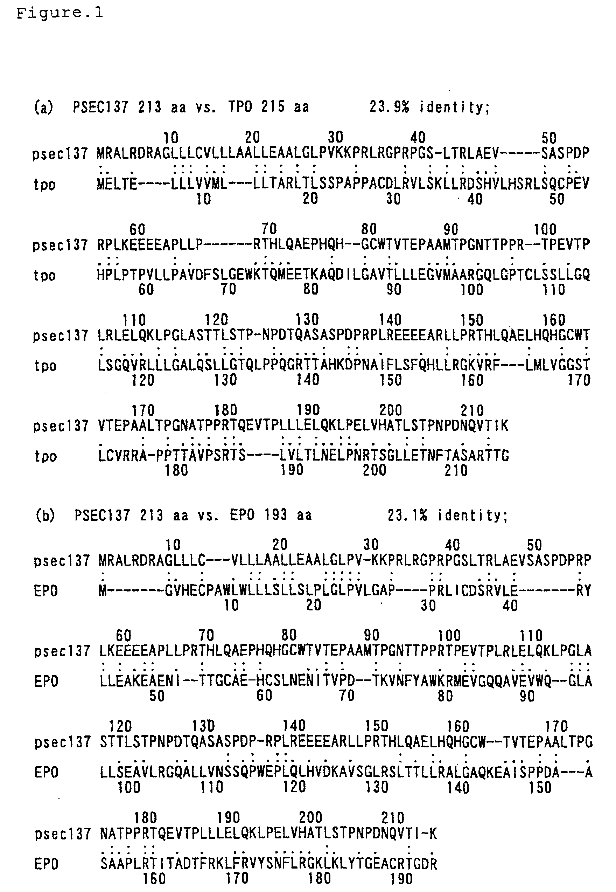

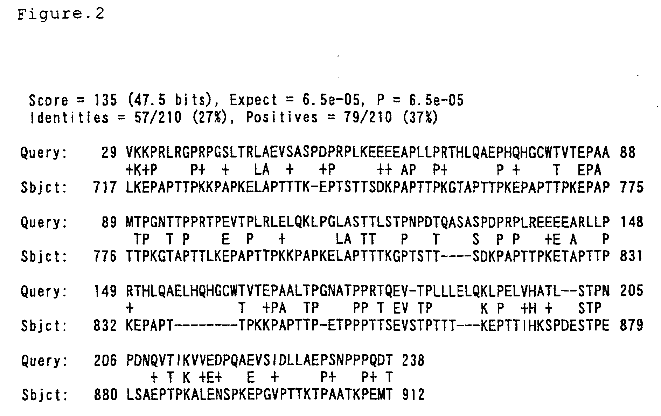

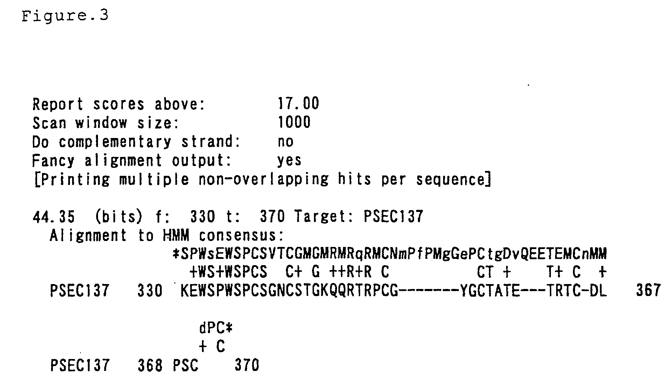

Through such analyses, the present inventors focused on PSEC0137 (hereinafter, referred to as PSEC137), which is one of the full-length cDNA clones. The 213 amino acids of N-terminal residues of the PSEC137 protein show 23.9% identity to the 215 amino acids of N-terminal residues of TPO comprising the TPO active fragment, and show 23.1% identity to EPO193 residues (Figure 1). A BLAST search of the non-redundant protein database indicated homology to megakaryocyte stimulating factor (Genbank Accession, U70136) (Figure 2). In the C-terminal region, a PFAM thrombospondin type 1 domain was identified (Figure 3). Repeating sequences that do not belong to known protein motifs exist on the PSEC137 protein sequence (amino acid residue numbers 47-127 and 128-208), and these sequences have 84% identity. Other proteins having structural communalities could not be found, and thus, PSEC137 was indicated as a novel protein.

Based on these facts, the present inventors found that the protein encoded by PSEC137 is a novel TPO/ EPO-like molecule, followed by completing the present invention. That is, the present invention relates to a novel secretory protein and its gene, as well as production and utilization for them as described below:
(1) a polynucleotide selected from the group consisting of (a) to (f) below:
   (a) a polynucleotide comprising a protein-coding region of the nucleotide sequence according to SEQ ID NO: 1;
   (b) a polynucleotide encoding a protein comprising the amino acid sequence according to SEQ ID NO: 2;
   (c) a polynucleotide encoding a protein that is functionally equivalent to a protein comprising the amino acid sequence according to SEQ ID NO: 2 in which one or more amino acids are replaced, deleted, inserted, and/or added;
   (d) a polynucleotide encoding a protein that is functionally equivalent to a protein comprising the amino acid sequence according to SEQ ID NO: 2, said polynucleotide hybridizing under stringent conditions with a polynucleotide comprising the nucleotide sequence according to SEQ ID NO: 1;
   (e) a polynucleotide encoding a partial peptide of a protein comprising the amino acid sequence according to SEQ ID NO: 2; and
   (f) a polynucleotide encoding a partial peptide of a protein that is functionally equivalent to a protein comprising the amino acid sequence according to SEQ ID NO: 2 in which one or more amino acids are replaced, deleted, inserted, and/or added;
(2) a protein encoded by the polynucleotide according to (1), or a partial peptide thereof;
(3) the partial peptide according to (2), said partial peptide comprising an amino acid sequence selected from the N-terminal amino acid residues 27 to 213 of SEQ ID NO: 2;
(4) a vector into which the polynucleotide according to (1) is inserted;
(5) a transformant comprising the polynucleotide according to (1) or the vector according to (4);
(6) a method for producing the protein or the partial peptide according to (2), said method comprising the steps of cultivating the transformant according to (5), and collecting expression products;
(7) a polynucleotide comprising at least 15 nucleotides, said polynucleotide hybridizing with the polynucleotide according to (1) or with a complementary strand thereof;
(8) a primer for synthesizing the polynucleotide according to (1), said primer comprising the polynucleotide according to (7);
(9) a probe for detecting the polynucleotide according to (1), said probe comprising the polynucleotide according to (7);
(10) an antisense DNA against the whole or a part of the polynucleotide according to (1);
(11) a method for isolating a gene encoding a receptor for the protein of (2), said method comprising the steps of:
   (a) contacting the protein according to (2) with a cell expressing a gene library; and
   (b) selecting a clone that can bind to the protein according to (2);
(12) a gene encoding a receptor for the protein according to (2), wherein said gene can be isolated by the method according to (11);
(13) a receptor for the protein according to (2), said receptor encoded by the gene according to (12);
(14) a method for screening a compound that interferes with binding between the protein according to (2) and a receptor for the protein, said method comprising:
   (a) contacting, with the protein according to (2), a cell that expresses a receptor for the protein according to (2), either in the presence of a candidate compound, or after said cell is contacted with a candidate compound; and
   (b) selecting a compound that interferes with a binding level of the protein according to (2);
(15) a compound interfering with binding between the protein according to (2) and a receptor for the protein, wherein said compound can be isolated by the method according to (14);
(16) a non-human vertebrate that has been manipulated so that expression of the protein according to (2) is altered;
(17) the non-human vertebrate according to (16), wherein said non-human vertebrate is a knockout animal or a transgenic animal; and
(18) the non-human vertebrate according to (17), wherein said non-human vertebrate is a mouse.

The "percent identity" of two amino acid sequences or of two nucleic acids is determined using the algorithm of Karlin and Altschul (Proc. Natl. Acad. Sei. USA 87:2264-2268, 1990), modified as in Karlin and Altschul (Proc. Natl. Acad. Sei. USA 90: 5873-5877, 1993).Such an algorithm is incorporated into the NBLAST and XBLAST programs of Altschul et al. (J. Mol. Biol. 215: 403-410, 1990). BLAST nucleotide searches are performed with the NBLAST program, score = 100, word length = 12. BLAST protein searches are performed with the XBLAST program, score = 50, word length = 3. When gaps exist between two sequences, Gapped BLAST is utilized as described in Altschul et al. (Nucleic Acids Res. 25: 3389-3402,1997). When utilizing BLAST and Gapped BLAST programs, the default parameters of the respective programs (e.g., BLASTX and BLASTN) are used. See http://www.ncbi.nlm.nih.gov.

The present invention relates to a novel secretory protein PSEC137. PSEC137 (SEQ ID NO: 2) included in the protein of the present invention is a secretory protein encoded by a gene that was obtained by screening cDNA prepared from human placental tissue. This protein is a novel growth and differentiation factor that has structural similarities to the homologous regions of EPO and TPO. Therefore, the protein of the present invention and its gene, as well as compounds of the present invention regulating the activity of the protein or expression of the genes may be applied to the prevention of and treatment for diseases caused by abnormalities of blood cells. In addition, causes of diseases may be elucidated by detecting abnormalities in the structures and expression levels of the genes and proteins of the present invention.

The protein of the present invention can be prepared as a recombinant protein or a natural protein. For example, a recombinant protein can be prepared by introducing a vector containing a DNA insert encoding the protein of the invention into an appropriate host cell, and purifying the expressed products from the transformant, as described below.

On the other hand, a natural protein can be prepared, for example, by utilizing an affinity column which is bound with the antibody against the protein of the invention, as described below ("Current Protocols in Molecular Biology" Ausubel et al. edit. (1987) John Wily & Sons, Section 16.1-16.19). The antibody used in the preparation of an affinity column can be a monoclonal antibody or polyclonal antibody. Alternatively, it is possible to prepare the protein of the invention by in vitro translation (See "On the fidelity of mRNA translation in the nuclease-treated rabbit reticulocyte lysate system." Dasso M.C., and Jackson R.J. (1989) Nucleic Acids Res. 17: 3129-3144).

The present invention includes a protein that is functionally equivalent to a protein comprising the amino acid sequence according to SEQ ID NO: 2, wherein the amino acid sequence of SEQ ID NO: 2 has been modified by replacement with other amino acids, deletion, insertion, and/or addition of one or more amino acids. "Functionally equivalent to a protein comprising the amino acid sequence according to SEQ ID NO: 2" means that the subject protein has similar biological characteristics to the PSEC137 protein. An example of biological characteristics of the PSEC137 protein is the activity to promote growth and differentiation by acting on hematopoietic progenitor cells. A protein having activity which is at least partially equivalent to the growth and differentiation promoting activity of the protein of the present invention can be considered as functionally equivalent.

In the present invention, a protein that is functionally equivalent to PSEC137 is desirable to show at least 85% or greater amino acid identity with the amino acid sequence of SEQ ID NO: 2. Specifically, the functionally equivalent protein of the present invention shows amino acid identity of 90% or greater, or more preferably 95% or greater. A BLAST search algorithm, and such can determine amino acid sequence identity.

There is no limitation on the number and sites of mutations in the amino acids of the protein as long as the function of the protein is maintained. Typically, the number of mutations is not more than 10% of all amino acids, preferably not more than 5% of all amino acids, and more preferably not more than 1% of all amino acids.

A protein that is functionally equivalent to PSEC137 can be prepared by one skilled in the art, for example, by a method to introduce mutations to the amino acid sequence in the protein (for example, site-directed mutagenesis (Current Protocols in Molecular Biology edit. Ausubel et al., 1987: Publish. John Wiley and Sons, Section 8.1-8.5). In addition, such proteins may be produced by naturally occurring amino acid mutations.

Whether the protein has the activity of a growth and differentiation factor can be confirmed by observing the growth and differentiation of cells expressing a receptor for the protein. A recombinant protein can be utilized as an affinity probe to identify the receptor-expressing cells. More specifically, the following methods can be exemplified:
(1) cultivating cells in which receptor expression has been identified, or cells that express known growth factors or its homologue in the presence of a candidate protein;
(2) observing the state of growth and differentiation of the cell, and comparing them to the results from a negative control or that in the presence of known growth and differentiation factors.

Growth stimulating activity of the candidate protein can be evaluated by determinating the number of cells, and by methods involving [³H]-thymidine uptake and such. In general, differentiation-inducing effect in hematopoietic progenitor cells is evaluated by investigating the effect on colony formation of progenitor cells. Such evaluation method is well known ("Colony Assays of Hematopoietic Cells Using Methylcellulose Media", An Introductory Technical Manual, Terry Fox Laboratory Media Preparation Service, Vancouver (1992)). In this evaluation, activity as a growth and differentiation factor can be detected more precisely by combining cytokines that act on hematopoietic cells such as IL-3, IL-6, or stem cell factor (SCF) as necessary.

Additionally, by administering the candidate proteins to laboratory animals (subcutaneously, intravenously, and so forth) and investigating their hematological parameters, biochemical profile, pathologic images, and such, the effect of a recombinant protein towards hematopoietic progenitor cells can be investigated. Furthermore, its function can be evaluated similarly by producing a transgenic animal that excessively expresses genes encoding a candidate protein.

In addition, by using a hybridization technology or a gene amplification technology well known to one skilled in the art a protein that is functionally equivalent to PSEC137 can be isolated. That is, one skilled in the art may ordinarily use a hybridization technique (Current Protocols in Molecular Biology edit. Ausubel et al., 1987: Publish. John Wiley and Sons, Section 6.3-6.4) to isolate DNA that is highly homologous to all or a part of the DNA sequence encoding PSEC137 (SEQ ID NO: 1), and then to obtain a protein that is functionally equivalent to this protein from the DNA. This way, the protein of this invention also comprises proteins encoded by DNA that hybridizes with DNA encoding PSEC137, that are functionally equivalent to these proteins.

Except humans, organisms to be isolated functionally equivalent proteins may include, for example, rat, rabbit, chicken, pig, cattle, and such, but are not limited to these examples.

The stringency of hybridization for isolating DNA encoding functionally equivalent proteins are normally at a level of "1x SSC, 0.1% SDS, and 37°C", more stringent conditions are at a level of "0.5x SSC, 0.1% SDS, and 42°C", and even more stringent conditions are at a level of "0.2x SSC, 0.1% SDS, and 65°C". As the hybridization conditions become more stringent, DNA that is highly homologous to the probe sequence is expected to be isolated. However, the above-mentioned combination of SSC, SDS, and temperature conditions provided as examples, and one skilled in the art can achieve similar stringency to those mentioned above by properly combining factors that determine the stringency of hybridization such as those mentioned above or others (for example, probe concentration, probe length, hybridization reaction time).

Proteins that are isolated by utilizing such hybridization technology usually have high amino acid sequence homology to PSEC137. High homology refers to at least 85% or higher, preferably 90% or higher, and even more preferably 95% or higher sequence identity.

In addition, a primer can be designed based on a part of the DNA sequence (SEQ ID NO: 1) encoding PSEC137 using gene amplification technology (PCR) (Current Protocols in Molecular Biology edit. Ausubel et al., 1987: Publish. John Wiley and Sons Section 6.1-6.4), then a DNA fragment having highly homologous to all or a part of the DNA sequence encoding PSEC137 can be isolated, followed by obtaining proteins that are functionally equivalent to the PSEC137 protein by using them.

The present invention also includes a partial peptide of the protein of the present invention. This partial peptide includes for example, a protein inwhich its signal peptide is removed. Furthermore, an antigenic peptide for antibody preparation is included. At least 7 amino acids, preferably 8 or more amino acids, and more preferably 9 or more amino acids of amino acid sequence should be comprised in the partial peptide for its specificity to the protein of the present invention. Well-known growth and differentiation factors such as TPO, is known to comprise a region that is conserved in all species in the N-terminal portion. Furthermore, this region is elucidated to have an important role for activity. Therefore, for the partial peptide of the present invention as well, partial peptide comprising amino acid sequence selected from the sequence containing the amino acids from positions 27 to 213 in the N-terminal has various utilities. Specifically, first, it is useful as an antigen for obtaining antibodies that can block the activity of the protein of the present invention. Secondly, it can provide an amino acid sequence of a synthetic peptide that functions as an agonist or an antagonist towards the protein of the present invention.

The peptide of the present invention can be used for preparing antibodies against the protein of the invention, or competitive inhibitors of them, and also screening for a receptor that binds to the protein of the invention. The partial peptides of the invention can be produced, for example, by genetic engineering methods, known methods for synthesizing peptides, or digesting the protein of the invention with an appropriate peptidase.

Furthermore, the present invention also relates to a polynucleotide encoding the protein of the invention. The polynucleotide of the invention can be provided in any form as far as it encodes the protein of the invention, and thus includes cDNA, genomic DNA, chemically synthesized DNA, and RNA, etc. The polynucleotide comprising any nucleotide sequence that is obtained based on the degeneracy of the genetic code is also included, as far as it encodes the protein of the invention. The polynucleotide of the invention can be isolated by the standard methods such as hybridization using a probe DNA comprising the nucleotide sequence encoding PSEC137 (SEQ ID NO: 1) or the portions thereof, or by PCR using primers that are-synthesized based on the nucleotide sequence.

The present invention also relates to a vector inserted the polynucleotide of the present invention. The vector of the invention is not limited as long as it contains the inserted polynucleotide stably. For example, if E. coli is used as a host, vectors such as pBluescript vector (Stratagene) are preferable as a cloning vector. To produce the protein of the invention, expression vectors are especially useful. Any expression vector can be used as far as it is capable of expressing the protein in vitro, in E. coli, in cultured cells, or in vivo. For example, pGEM vector (Promega) is preferable for in vitro expression, pET vector (Novagen) for E. coli, pME18S-FL3 vector (GenBank Accession No. AB009864) for cultured cells, and pME18S vector (Mol. Cell. Biol. (1988) 8: 466-472) for in vivo expression to insert the polynucleotide of the invention, ligation utilizing restriction sites can be performed according to the standard method (Current Protocols in Molecular Biology (1987) Ausubel et al. edit, John Wily & Sons, Section 11.4-11.11).

In addition, the present invention also relates to a transformant carrying the polynucleotide or the vector of the invention. Any cell can be used as a host into which the vector of the invention is inserted, and various kinds of host cells can be used depending on the purposes. For strong expression of the protein in eukaryotic cells, COS cells or CHO cells can be used, for example. Similarly to TPO, the protein of the present invention comprising the amino acid sequence according to SEQ ID NO: 2 has a structure in which several bindings of sugar chains are predicted,. The Asn-X-[Ser, Thr] tripeptide (wherein, X indicates any amino acid, [Ser, Thr] indicates one of Ser or Thr) corresponding to a site capable of N-type sugar chain modification exists at five positions on the amino acid sequence, and those sites correspond to amino acid positions 93, 174, 300, 341, and 392. Therefore, if eukaryotic cells are used for the expression of a protein comprising the amino acid sequence according to SEQ ID NO: 2, a molecule with sugar chains added to it can be obtained. Such molecules are thought to be structurally similar form to the naturally occurring form. Therefore, the method using eukaryotic cells as an expression host constitutes a preferred embodiment of the present invention. In particular, mammalian cells, such as COS cells, and CHO cells are preferred for eukaryotic cells.

Insertion of a vector into a host cell can be carried out by methods such as calcium phosphate precipitation, electroporation (Current Protocols in Molecular Biology edit. Ausubel et al., 1987: Publish. John Wiley and Sons, Section 9.1-9.9), lipofectamine (Gibco-BRL), and microinjection.

In addition, the present invention relates to a polynucleotide having at least 15 nucleotides length, and specifically hybridizes with the polynucleotide of SEQ ID NO: 1. "Specifically hybridize" with the polynucleotide of the present invention means that, under normal hybridization conditions, or preferably under stringent conditions, hybridizing with the polynucleotide of the present invention but not with other polynucleotide. Such polynucleotide can be used as a probe for isolation and detection of the polynucleotide of the invention, and as a primer for amplifying the polynucleotide of the present invention. As a primer, it usually has a length of 15-100 bp, preferably 15-50 bp, and more preferably has a length of 15-35 bp. As a probe, it contains the entire sequence of the polynucleotide of the invention, or at least the portion of it, and has a length of at least 15 bp.

The polynucleotide of the present invention can be used for examination and diagnosis of the abnormality of the protein of the invention. For example, it is possible to examine the abnormal expression of the gene encoding the protein using the polynucleotide of the invention as a probe for Northern hybridization or as a primer for RT-PCR. Also, the polynucleotide of the invention can be used as a primer for polymerase chain reaction (PCR) such as the genomic DNA-PCR, and RT-PCR to amplify the polynucleotide encoding the protein of the invention, or the regulatory region of the expression, with which it is possible to examine and diagnose the abnormality of the sequence by RFLP analysis, SSCP, and direct sequencing, etc.

The "polynucleotide of the present invention having a length of at least 15 nucleotides, specifically hybridizing with a polynucleotide described in SEQ ID NO: 1", includes an antisense DNA for suppressing the expression of the protein of the invention. To exert the antisense effect, the antisense DNA has a length of at least 15 bp or more, preferably 100 bp, and more preferably 500 bp or more, and has a length of usually 3000 bp or less and preferably 2000 bp or less. The antisense DNA can be used in the gene therapy of the diseases that are caused by the abnormality of the protein of the invention (abnormal function or abnormal expression) (specifically, the diseases associated with the abnormality of the hemocytes). Said antisense DNA can be prepared, for example, by the phosphorothioate method ("Physicochemical properties of phosphorothioate oligodeoxynucleotides." Stein (1988) Nucleic Acids Res, 16: 3209-3221) based on the nucleotide sequence of the DNA encoding the protein (for example, the DNA described in SEQ ID NO: 1).

The polynucleotide or antisense DNA of the present invention can be used in gene therapy, for example, by administrating it into a patient by the in vivo or ex vivo method with virus vectors such as retrovirus vectors, adenovirus vectors, and adeno-associated virus vectors, or non-virus vectors such as liposome.

The present invention also relates to antibodies that bind to the protein of the invention. There are no limitations in the form of the antibodies of the invention. They include polyclonal antibodies, monoclonal antibodies, or their portions that can bind to the antigen. They also include antibodies of all classes. Furthermore, special antibodies such as humanized antibodies are also included.

The polyclonal antibody of the invention can be obtained according to the standard method by synthesizing an oligopeptide corresponding to the amino acid sequence and immunizing rabbits with the peptide (Current Protocols in Molecular Biology (1987) Ausubel et al. edit, John Wily & Sons, Section 11.12-11.13). The monoclonal antibody of the invention can be obtained according to the standard method by purifying the protein expressed in E. coli, immunizing mice with the protein, and producing a hybridoma cell by fusing the spleen cells and myeloma cells (Current Protocols in Molecular Biology (1987) Ausubel et al. edit, John Wily & Sons, Section 11.4-11.11).

The antibody binding to the protein of the present invention can be used for purification of the protein of the invention, and also for detection and/or diagnosis of the abnormalities of the expression and structure of the protein. Specifically, proteins can be extracted, for example, from tissues, blood, or cells, and the protein of the invention is detected by Western blotting, immunoprecipitation, or ELISA, etc. for the above purpose.

Furthermore, the antibody binding to the protein of the present invention can be utilized for treating the diseases that associates with the protein of the invention. If the antibodies are used for treating patients, human antibodies or humanized antibodies are preferable in terms of their low antigenicity. The human antibodies can be prepared by immunizing a mouse whose immune system is replaced with that of human ("Functional transplant of megabase human immunoglobulin loci recapitulates human antibody response in mice" Mendez M.J. et al. (1997) Nat. Genet. 15: 146-156, for a reference). The humanized antibodies can be prepared by recombination of the hyper variable region of a monoclonal antibody (Methods in Enzymology (1991) 203: 99-121).

In addition, the present invention relates to a method for isolating a gene encoding a receptor that binds to the protein of the present invention by using the protein of the present invention. This isolation method is based on the so-called expression cloning principle. That is, the method for isolating the receptor based on the present invention comprises (a) contacting the protein of the present invention with the cells that express a gene library, and (b) selecting a clone that can bind to the protein of the present invention.

Furthermore, the present invention relates to a receptor protein encoded by genes that can be obtained by this method. The protein of the present invention and its receptor, as well as genes encoding them are useful for screening of compounds that interfere with the binding between them. Alternatively, this receptor may be utilized for isolating a homologue of a protein comprising the amino acid sequence of SEQ ID NO: 2.

There is no limitation on the gene library used for above isolation method as long as it has the possibility of containing genes of the receptor. For such a library, a cDNA library derived from, for example, various blood cells or their progenitor cells may be used. More specifically, cDNA library derived from hematopoietic stem cells, megakaryocytic progenitor cells, even differentiated megakaryocytic progenitor cells such as promegakaryoblastic, megakaryoblastic, promegakaryocytic, megakaryocytic cell, and such may be used. A method for preparing cDNA from these cells, followed by preparing an expression library of the cDNA is well known.

It is advantageous to use a labeled protein for selecting a clone as a ligand that binds to the protein of the present invention. For example, by expressing the protein of the present invention as a fusion protein with a fluorescent protein such as GFP, it can be made into a fluorescence-labeled ligand. Alternatively, when expressed as a fusion protein with an immunological tag such as a myc tag, the ligand may be traced by using antibodies against the tag. A gene encoding the receptor of the protein of the present invention can be isolated by above-described selection and isolation of a clone that binds to a ligand.

The isolated receptor may be used for screening substances that interfere with binding to the protein of the present invention. That is, the present invention provides a method for screening compounds that interfere with binding between the protein of the present invention and its receptor, comprising:
(a) contacting the cell expressing above receptor with the protein of the present invention either in the presence of a candidate compound or after contacting above cell with the candidate compound; and
(b) selecting a compound that interferes with the binding level of the protein of the present invention.

As cells to express above receptor, naturally-occuring cells known to express above receptor may be used. Alternatively, cells that do not express above receptor by nature, and that transformed by the gene encoding it may be used. Binding of the protein of the present invention to a cell expressing above receptor may be confirmed readily by labeling the protein. The protein of the present invention maybe labeled with fluorescent proteins such as GFP, various enzymes, and immunological tags using well-known methods.

By the screening method of the present invention, a partial peptide that binds to a receptor in a similar manner to the protein of the present invention can be obtained. Similarly to TPO/ EPO, such a partial peptide may have the activity to function as a differentiation and growth factor towards hematopoietic cells, and can be expected to be a therapeutic agent. Or conversely, compounds that inhibit binding of the protein of the present invention to the receptor, and do not accompany signal transduction, are useful as research materials to further elucidate the function of the protein of the present invention.

When using a compound isolated by the screening method of the present invention as a therapeutic agent, the isolated compound may be formulated for administration by well-known pharmaceutical methods, as well as directly administered itself to the patient. For example, it may be combined suitably with pharmacologically acceptable carrier or medium, specifically, with sterilized water, saline, vegetable oil, emulsifier, suspension, and such for formulation and administration. Administration to patients may be carried out by methods that are well known to one skilled in the art, for example, intraarterial injection, intravenous injection, subcutaneous injection, and such. Although dosage changes depending on the age or weight of the patient, and on the method for the administration, one skilled in the art can properly select the appropriate dosage. In addition, if DNA can encode the compound, the DNA can be inserted into a gene therapeutic vector for gene therapy. Although dosage and the method of administration will change depending on the weight, age, and symptom of the patient, by one skilled in the art can properly select them.

Additionally, the present invention provides a non-human vertebrate that is manipulated so that expression of the protein of the present invention is altered. Herein, "alteration of the expression" includes enhancement as well as attenuation of expression. Furthermore, "Alteration of expression of the protein" includes alterations in both transcription and translation steps. Such non-human vertebrates include animals that are manipulated to stop or decrease expression of endogenous proteins of the present invention ("knockout animals"), and animals that are inserted genes encoding exogenous proteins to express the proteins of the present invention ("transgenic animals"). These knockout and transgenic non-human vertebrates can be prepared following the publication, "Neuroscience Laboratory Manual 3, Gene Manipulation of Embryos and Individuals for Neurobiology (edit. Kondo, T., Springer Verlag, Tokyo)".

For example, by using a produced transgenic animal in which DNA encoding the PSEC137 protein of the present invention is inserted into its chromosome, the expression of these proteins can be elevated or their distribution and expression pattern can be altered. In addition, by introducing a mutation into the expression regulating region of these endogenous genes, or by adding or replacing other expression regulation regions, transcription level can be artificially elevated or diminished comparing to the originally expression level of the genes, or the expression pattern and distribution can be altered. On the other hand, deletion of a portion of the exon, or substitution with a stop codon due to introduction of a point mutation to the open reading frame, can modify the translation leading to the protein. Additionally, by expression of antisense RNA and ribozymes, expression of the PSEC137 gene can be regulated. Introduction of these mutations can be carried out by well-known methods.

These non-human vertebrates are useful for studying the function of transcription, elucidating the mechanism of diseases relating to transcription, and developing animal disease models to be used for screening therapeutic agents, and such.

### Brief Description of the Drawings

Figure 1 is a diagram that shows the results of comparing the amino acid sequence of the PSEC137 protein of the present invention with the amino acid sequence of the known TPO protein (a), and EPO protein (b). The symbol ": " indicates identical amino acids, and "." indicates homologous amino acids.
Figure 2 is a diagram that shows the results of comparing the amino acid sequence of the PSEC137 protein of the present invention with that of megakaryocyte stimulating factor (Genbank Accession No: U70136). When both constituent amino acids were common, one letter code indicating that amino acid was indicated. Homologous amino acids were indicated by a "+" sign.
Figure 3 is a diagram that shows the PFAM thrombospondin type 1 domain found in the C-terminal region in the amino acid sequence of the PSEC137 protein of the present invention.
Figure 4 is a photograph showing the results of analyzing the topography of the PSEC137 gene. The results of Northern blotting are shown in (a), and the results of RT-PCR are shown in (b).
Figure 5 is a photograph showing the results of purifying the PSEC137 protein by thio-affinity purification. The estimated molecular weight of the PSEC137 fusion protein predicted from its amino acid sequence is 78.6 kDa.

### Best Mode for Carrying out the Invention

The present invention will explain in detail below with reference to examples, however it is not to be construed as being limited thereto. [Example 1] Isolation of PSEC137

Human placenta tissues were used to extract mRNA by the method described in the literature (Molecular Cloning 2nd edition. Sambrook J., Fritsch, E.F., and Maniatis T. (1989) Cold Spring Harbor Laboratory Press). Furthermore, poly(A)⁺RNA was purified from the mRNA using oligo-dT cellulose.

Poly(A)⁺RNA was used to construct a cDNA library by the oligo-capping method (Maruyama M. and Sugano S. (1994) Gene 138: 171-174).

Using the Oligo-cap linker (agcaucgagu cggccuuguu ggccuacugg/SEQ ID NO: 3) and the Oligo-dT primer (gcggctgaag acggcctatg tggccttttt tttttttttt tt/SEQ ID NO: 4), BAP (bacterial alkaline phosphatase) treatment, TAP (tobacco acid phosphatase) treatment, RNA ligation, the first strand cDNA synthesis, and removal of RNA were performed as described in the reference (Suzuki and Kanno (1996) Protein Nucleic acid and Enzyme. 41: 197-201; Suzuki Y. et al. (1997) Gene 200: 149-156). Next, 5'-PCR primer (agcatcgagt cggccttgtt g/SEQ ID NO: 5) and 3'-PCR primer (gcggctgaag acggcctatg t/SEQ ID NO: 6) were used for performing PCR (polymerase chain reaction) to convert the cDNA into double stranded cDNA, which was then digested with SfiI. Then, the DraIII-cleaved pME18SFL3 was used for cloning the cDNA in an unidirectional manner, and cDNA libraries were obtained. The clones having an insert cDNA with a length of 1 kb or less were discarded. Then, the nucleotide sequence of the 5' and 3' ends of the cDNA clones was analyzed with a DNA sequencer (ABI PRISM 377, PE Biosystems) after sequencing reactions were performed with the DNA sequencing reagents (Dye Terminator Cycle Sequencing FS Ready Reaction Kit, dRhodamine Terminator Cycle Sequencing FS Ready Reaction Kit, or BigDye Terminator Cycle Sequencing FS Ready Reaction Kit, from by PE Biosystems) according to the instructions.

The fullness ratio at the 5'-end sequences of the cDNA clones in the libraries constructed by the oligo-capping method was determined as follows. Of all the clones whose 5'-end sequences were found in those of known human mRNA in the public database, a clone was judged to be "full-length", if it had a longer 5'-end sequence than that of the known human mRNA, or, even though the 5'-end sequence was shorter, if it contained the translation initiation codon. A clone that did not contain the translation initiation codon was judged to be "non-full-length". The fullness ratio ((the number of full-length clones)/(the number of full-length and non-full-length clones)) at the 5' end of the cDNA clones from each library was determined by comparing with the known human mRNA. The results indicates that the fullness ratio of the library was 62%, and that the fullness ratio at the 5'-end sequence was extremely high.

Next, the fullness ratio at the 5' end of the cDNA was estimated by using the ATGpr and the ESTiMateFL.

The ATGpr, developed by SalamovA.A., Nishikawa T., and Swindells M.B. in the Helix Research Institute, is a program for prediction of the translation initiation codon based on the characteristics of the sequences in the vicinity of the ATG codon [A. A. Salamov, T. Nishikawa, M. B. Swindells, Bioinformatics, 14: 384-390 (1998); http://www.hri.co.jp/atgpr/]. The results are shown with expectations (also described as ATGpr1 below) that an ATG is a true initiation codon (0.05-0.94). The results indicate that the ATGpr1 value of PSEC137 was 0.94.

The ESTiMateFL, developed by Nishikawa and Ota in the Helix Research Institute, is a method for the selection of a clone with high fullness ratio by comparing with the 5'-end or 3'-end sequences of ESTs in the public database.

By the method, a cDNA clone is judged presumably not to be full-length if there are any ESTs that have longer 5'-end or 3'-end sequences than the clone. The method is systematized for high throughput analysis. A clone is judged to be full-length if the clone has a longer 5' -end sequence than ESTs in the public database. Even if a clone has a shorter 5' end, the clone is judged to be full-length if the difference in length is within 50 bases, and otherwise judged not tobe full-length, for convenience. The precision of the prediction by comparing cDNA clones with ESTs is improved with increasing number of ESTs to be compared. However, when only a limited number of ESTs are available, the reliability becomes low. Thus, the method is effective in excluding clones with high probability of being not-full-length, from the cDNA clones that is synthesized by the oligo-capping method and that have the 5'-end sequences with about 60 % fullness ratio. In particular, the ESTiMateFL is efficiently used to estimate the fullness ratio at the 3'-end sequence of cDNA of a human unknown mRNA that has a significant number of ESTs in the public database.

Next, using a protein localization-predicting program "PSORT" (K. Nakai and M. Kanehisa, Genomics, 14: 897-911 (1992)), developed by Nakai and Kanehisa, the presence of sequences estimated to be signal peptides characteristic of the amino termini of many secretory proteins was analyzed for all the deduced amino acid sequences initiating from each ATG codon in the 5' -end sequence of clones of the library prepared bytheoligo-cappingmethod. Thus, clones estimated to comprise signal sequences (most likely to be secretory proteins or membrane proteins) were specifically selected from the clones of the library prepared by the oligo-capping method. As a result, it has been predicted that PSEC137 is a secretory protein or a membrane protein, has a signal sequence at its N-terminus, and is a full-length cDNA clone.

For the clone selected as above, the nucleotide sequences of the full-length cDNA and the deduced amino acid sequences were further determined. The nucleotide sequences were finally determined by overlapping completely the partial nucleotide sequences determined by the following three methods.
(1) Long-read sequencing from both ends of the cDNA inserts using a Licor DNA sequencer (After sequence reactions were performed according to the manual for the Licor sequencer (Aroka), DNA sequence was determined by the sequencer.)
(2) Nested sequencing by the Primer Island method which utilizes the in vitro transfer of AT2 transposon (Devine S.E., and Boeke J.D. (1994) Nucleic Acids Res. 22: 3765-3772) (After clones were obtained using a kit from PE Biosystems, sequence reactions were performed using the DNA sequencing reagents from the company, according to the manufacturer's instructions, and DNA sequence was determined using an ABI PRISM 377 sequencer.)
(3) Primer walking by the dideoxy terminator method using custom synthesized DNA primers (After sequence reactions were performed using the DNA sequencing reagents from PE Biosystems and custom synthesized DNA primers according to the manufacturer' s instructions, DNA sequence was determined using an ABI PRISM 377 sequencer).

These sequences were subjected to the analysis by the ATGpr and PSORT and also to the BLAST search of the GenBank and SwissProt. As a result, it has been predicted that PSEC137 is a secretory protein or a membrane protein, has a signal sequence at its N-terminus, and is a full-length cDNA clone.

### [Example 2] Protein Homology Analysis

For the expected amino acid sequence of the PSEC137 protein, motif search and homology analysis to known proteins were performed. The isolated PSEC137 cDNA encodes a protein comprising 571 amino acid residues (SEQ ID NO: 2). Using a program for predicting signal sequences and protein localization, PSORT (Trends Biochem Sci. 1999 Jan; 24 (1): 34-36), it has been predicted that PSEC137 is a secretory protein that has a signal sequence of 26 amino acid residues and whose mature form has 545 amino acid residues (amino acid residues 27 to 571). Since a search of Blocks library (Nucl. Acids Res. 27:226-228 (1999)) identified erythropoietin (EPO)/ thrombopoietin (TPO) protein-like sequence fragment (BL00817) with a low score, a pair-wise sequence comparison was performed between the PSEC137 protein and human EPO and TPO, respectively (SwissProt Accession Nos. were P01588 arid P40225, respectively).

The 213 amino acids of N-terminal residues of the PSEC137 protein show 23.9% identity to the 215 amino acids of N-terminal residues of TPO comprising the TPO active fragment, and show 23.1% identity to EPO193 residues (Figure 1). A BLAST search of the non-redundant protein database indicated homology to megakaryocyte stimulating factor (Genbank Accession, U70136) (Figure 2). In the C-terminal region, a PFAM thrombospondin type 1 domain was identified (Figure 3). Repeating sequences that do not belong to known protein motifs exist on the PSEC137 protein sequence (amino acid residue numbers 47-127 and 128-208), and these sequences have 84% identity.

### [Example 3] Gene Expression Topography

The expression topography of the PSEC137 gene was analyzed by Northern blotting and RT-PCR. The PSEC137 gene StuI fragment (243bp) was excised, and was labeled with ³²P-dCTP using RTG DNA Labelling Beads (dCTP) (Amersham Pharmacia Biotech) to prepare probes. Using a filter on which mRNAs derived from 12 human tissues was blotted (Human 12-Lane MTN Blot; Clontech), hybridization was performed in ExpressHyb hybridization solution (Clontech) according to the manufacturer's directions, and then the probe was washed off under high-stringency conditions directed by the manufacturer.

Using Human MTC Panel (Clontech), tissue expression analysis was performed by RT-PCR. The primers used for amplification are as follows: hPSEC137FOR: GCTTCTGCCTGCGTTCCATGCTGTCTG (SEQ ID NO: 7); and

hPSEC137REV: GGCACACAGCCTCGGACCAACCTCACT (SEQ ID NO: 8).

As heat-resistant DNA polymerase for PCR, AmpliTaq Gold (PE Applied Biosystems) was selected to prepare the reaction solution according to the manufacturer's directions. The final concentration of the primer was 200 nM. The reaction cycle was 94°C for 10 min, followed by 40 cycles of 94°C for 30 sec, 55°C for 30 sec, and 72°C for 30 sec.

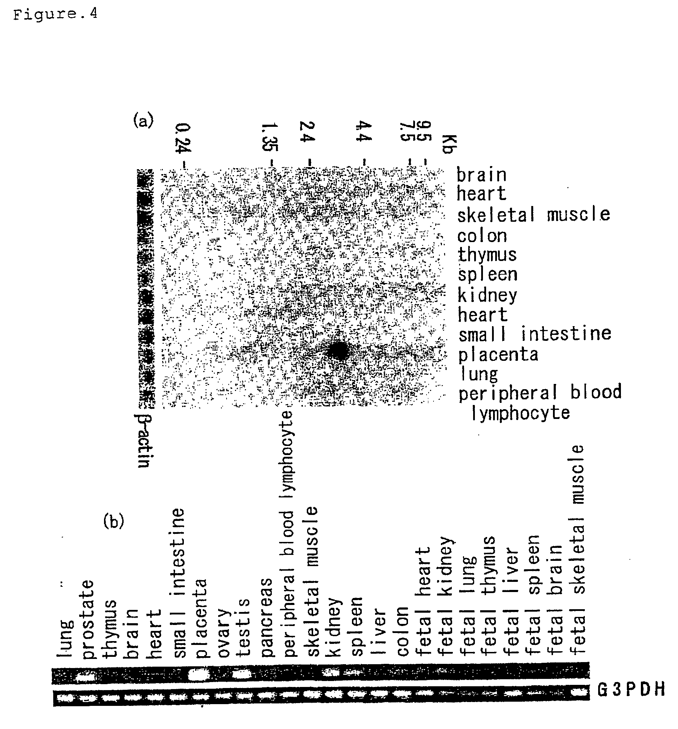

The results of Northern blotting (a) , and the results of RT-PCR (b) are shown in Figure 4. A transcription product of approximately 3.0 kb in the placenta was detected on Northern blotting. The resulting size is not contradictory to the cloned full-length genetic sequence described in Example 1. Strong gene expression is localized in the placenta, and weak expression was observed in the prostate, testis, kidney (including those in the embryonic stage), and spleen. Since its expression is strong and localized at the placenta, PSEC137 product is suggested to involve in maintenance of pregnancy, and maintenance of embryonic growth.

### [Example 4] Preparation of the PSEC137 Protein

Recombinant PSEC137 protein can be produced by a variety of expression systems. For example, PSEC137 lacking the signal sequence can be expressed as a recombinant thioredoxin fusion protein. PSEC137 structural gene lacking the signal sequence was inserted into pET-32a (Novagen) to construct an expression vector. The PSEC137 gene was amplified by PCR using two primers, 5'-ctccccgtgaagaagccgcggctc-3' (SEQ ID NO: 9) and 5'-gcaagcttctagtactccttggcctcctgcaa-3' (SEQ ID NO: 10), digested with HindIII, and cloned into pET32a that is digested with EcoRV and HindIII. BL21 (DE3) trxB strain was transformed with the prepared expression vector, followed by induction of its expression by addition of isopropyl β-D(-) -thiogalactopyranoside. Introducing the expression at a cultivation temperature of 30°C enabled recovery of approximately 50% of the protein as a soluble protein.

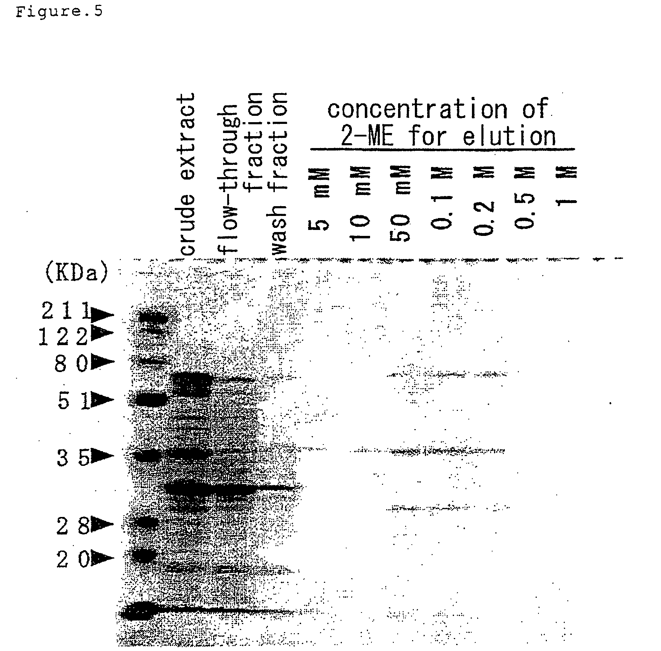

A purification procedure using soluble fractions is indicated as follows. Cultivation (100 mL) was carried out at 27°C. After induced expressing, the culture fluid was further cultivated and then centrifuged, and the resulting pellet was stored in a freezer at -80°C. After thawed on ice, the pellet was suspended in 5 mL of bacterial protein extraction solution, B-PER (Pierce), containing protease inhibitors. Upon standing at room temperature for 10 minutes, the mixture was centrifuged, and the supernatant was filtered through a 22-µm-pore membrane, and then subjected to the affinity purification using thioredoxin (ThioBond Resin: Invitrogen). Binding was carried out in batches (1 mL of resin), and then the resulting mixture was packed into a column, and was washed with Tris buffer saline (pH 7.4) containing 1 mM 2-mercaptoethanol (2-ME). Subsequently, the resulting solution was eluted with 3 mL each of 5, 10, 50, 100, 200, 500, and 1000 mM 2-mercaptoethanol (2-ME), and each collected fractions were analyzed by SDS-PAGE and Western blotting. The PSEC137 fusion protein of interest could be purified partially by binding to the resin according to the above-mentioned conditions, and then by being eluted with 50 to 200 mM 2-ME (Figure 5).

### Industrial Applicability

The present invention provides a novel PSEC137 protein and a gene encoding it. PSEC137 is a protein comprising a TPO/ EPO-like amino acid sequence. Therefore, this protein is useful as a growth and differentiation factor. For example, differentiation and growth activity of the protein of the present invention for blood cells can be expected. By using this activity, a novel therapeutic agent having hematopoietic activity can be produced. On the other hand, the gene of the present invention is useful for the production of the protein.

Alternatively, the protein of the present invention can be used as a ligand to obtain novel receptors for growth and differentiation factors of blood cells.

## Claims

1. A polynucleotide selected from the group consisting of (a) to (f) below:
(a) a polynucleotide comprising a protein-coding region of the nucleotide sequence according to SEQ ID NO: 1;
(b) a polynucleotide encoding a protein comprising the amino acid sequence according to SEQ ID NO: 2;
(c) a polynucleotide encoding a protein that is functionally equivalent to a protein comprising the amino acid sequence according to SEQ ID NO: 2 in which one or more amino acids are replaced, deleted, inserted, and/or added;
(d) a polynucleotide encoding a protein that is functionally equivalent to a protein comprising the amino acid sequence according to SEQ ID NO: 2, said polynucleotide hybridizing under stringent conditions with a polynucleotide comprising the nucleotide sequence according to SEQ ID NO: 1;
(e) a polynucleotide encoding a partial peptide of a protein comprising the amino acid sequence according to SEQ ID NO: 2; and
(f) a polynucleotide encoding a partial peptide of a protein that is functionally equivalent to a protein comprising the amino acid sequence according to SEQ ID NO: 2 in which one or more amino acids are replaced, deleted, inserted, and/or added.

2. A protein encoded by the polynucleotide according to claim 1, or a partial peptide thereof.

3. The partial peptide according to claim 2, said partial peptide comprising an amino acid sequence selected from the N-terminal amino acid residues 27 to 213 of SEQ ID NO: 2.

4. A vector into which the polynucleotide according to claim 1 is inserted.

5. A transformant comprising the polynucleotide according to claim 1 or the vector according to claim 4.

6. A method for producing the protein or the partial peptide according to claim 2, said method comprising the steps of cultivating the transformant according to claim 5, and collecting expression products.

7. A polynucleotide comprising at least 15 nucleotides, said polynucleotide hybridizing with the polynucleotide according to claim 1 or with a complementary strand thereof.

8. A primer for synthesizing the polynucleotide according to claim 1, said primer comprising the polynucleotide according to claim 7.

9. A probe for detecting the polynucleotide according to claim 1, said probe comprising the polynucleotide according to claim 7.

10. An antisense DNA against the whole or a part of the polynucleotide according to claim 1.

11. A method for isolating a gene encoding a receptor for the protein of claim 2, said method comprising the steps of:
(a) contacting the protein according to claim 2 with a cell expressing a gene library; and
(b) selecting a clone that can bind to the protein according to claim 2.

12. A gene encoding a receptor for the protein according to claim 2, wherein said gene can be isolated by the method according to claim 11.

13. A receptor for the protein according to claim 2, said receptor encoded by the gene according to claim 12.

14. Amethod for screening a compound that interferes with binding between the protein according to claim 2 and a receptor for the protein, said method comprising:
(a) contacting, with the protein according to claim 2, a cell that expresses a receptor for the protein according to claim 2, either in the presence of a candidate compound, or after said cell is contacted with a candidate compound; and
(b) selecting a compound that interferes with a binding level of the protein according to claim 2.

15. A compound interfering with binding between the protein according to claim 2 and a receptor for the protein, wherein said compound can be isolated by the method according to claim 14.

16. A non-human vertebrate that has been manipulated so that expression of the protein according to claim 2 is altered.

17. The non-human vertebrate according to claim 16, wherein said non-human vertebrate is a knockout animal or a transgenic animal.

18. The non-human vertebrate according to claim 17, wherein said non-human vertebrate is a mouse.


SEQUENCE LISTING

<110> Helix Research Institute

<120> GROWTH AND DIFFERENTIATION FACTOR

<130> H1-106PCT3

<140>
<141>

<150> JP 1999-194179
<151> 1999-07-08

<150> US 60/159586
<151> 1999-10-18

<160> 10

<170> PatentIn Ver. 2.0

<210> 1
<211> 2981
<212> DNA
<213> Homo sapiens

<220>
<221> CDS
<222> (58)..(1770)

<400> 1
gactgggttc gcggccgcgt gcagaggtgc aggcagagca gcctcggaac cgagacg 57

atg cgt gcg ctc cgc gac cga gcc ggg ctc ctc ctc tgc gtg ctg ctg 105
Met Arg Ala Leu Arg Asp Arg Ala Gly Leu Leu Leu Cys Val Leu Leu
1               5                   10                  15

ctg gcg gcg ctg ctg gag gcg gcg cta ggg ctc ccc gtg aag aag ccg 153
Leu Ala Ala Leu Leu Glu Ala Ala Leu Gly Leu Pro Val Lys Lys Pro
            20                  25                  30

cgg ctc cgc gga cca cgg cct ggg agc ctc acg agg ctc gca gag gtc 201
Arg Leu Arg Gly Pro Arg Pro Gly Ser Leu Thr Arg Leu Ala Glu Val
        35                  40                  45

tca gcc tcc cca gat cct agg cct ctg aag gaa gag gag gag gca cca 249
Ser Ala Ser Pro Asp Pro Arg Pro Leu Lys Glu Glu Glu Glu Ala Pro
    50                  55                  60

ctg ctc ccc aga acc cac ctg cag gca gag cca cac caa cat gga tgc 297
Leu Leu Pro Arg Thr His Leu Gln Ala Glu Pro His Gln His Gly Cys
65                  70                  75                  80

tgg act gtc act gag cca gca gcc atg acc cca ggc aac acc acc cct 345
Trp Thr Val Thr Glu Pro Ala Ala Met Thr Pro Gly Asn Thr Thr Pro
                85                  90                  95

ccc agg acc cca gag gtt act ccg ttg cgg ctg gag ctg cag aag ctg 393
Pro Arg Thr Pro Glu Val Thr Pro Leu Arg Leu Glu Leu Gln Lys Leu
            100                 105                 110

ccg gga ttg gcc agc aca acc ttg agt acc cct aac cct gat acc cag 441
Pro Gly Leu Ala Ser Thr Thr Leu Ser Thr Pro Asn Pro Asp Thr Gln
        115                 120                 125

gct tca gcc tcc cca gat cct agg cct ctg agg gaa gag gag gag gca 489
Ala Ser Ala Ser Pro Asp Pro Arg Pro Leu Arg Glu Glu Glu Glu Ala
    130                 135                 140

cga ctg ctc ccc aga acc cac ctg cag gca gag cta cac caa cat gga 537
Arg Leu Leu Pro Arg Thr His Leu Gln Ala Glu Leu His Gln His Gly
145                 150                 155                 160

tgt tgg act gtc act gag cca gca gcc ctg acc cca ggg aat gcc acg 585

Cys Trp Thr Val Thr Glu Pro Ala Ala Leu Thr Pro Gly Asn Ala Thr
                165                 170                 175

cct ccc agg acc cag gag gtt act ccc ttg ctg ctg gag ctg cag aag 633
Pro Pro Arg Thr Gln Glu Val Thr Pro Leu Leu Leu Glu Leu Gln Lys
            180                 185                 190

ctg cca gaa ttg gtc cac gca acc ttg agt acc cct aac cct gat aac 681
Leu Pro Glu Leu Val His Ala Thr Leu Ser Thr Pro Asn Pro Asp Asn
        195                 200                 205

cag gtg acc atc aag gtg gtg gag gac ccc cag gcc gag gtg tcg ata 729
Gln Val Thr Ile Lys Val Val Glu Asp Pro Gln Ala Glu Val Ser Ile
    210                 215                 220

gac ctg ttg gct gag ccc agc aat ccc ccg ccc cag gat acc ctt agc 777
Asp Leu Leu Ala Glu Pro Ser Asn Pro Pro Pro Gln Asp Thr Leu Ser
225                 230                 235                 240

tgg ctg ccc gcc ctc tgg ccc ttc ctc tgg gga gac tac aaa gga gag 825
Trp Leu Pro Ala Leu Trp Pro Phe Leu Trp Gly Asp Tyr Lys Gly Glu
                245                 250                 255

gaa aaa gac agg gcc cca ggg gag aag ggg gag gaa aag gag gaa gac 873
Glu Lys Asp Arg Ala Pro Gly Glu Lys Gly Glu Glu Lys Glu Glu Asp
            260                 265                 270

gag gac tat cct tca gag gat atc gag ggt gag gat caa gag gac aaa 921
Glu Asp Tyr Pro Ser Glu Asp Ile Glu Gly Glu Asp Gln Glu Asp Lys
        275                 280                 285

gag gaa gat gag gaa gag cag gcg ctc tgg ttc aat gga act aca gac 969
Glu Glu Asp Glu Glu Glu Gln Ala Leu Trp Phe Asn Gly Thr Thr Asp
    290                 295                 300

aac tgg gac cag ggc tgg ctg gcc ccc ggg gat tgg gtc ttc aag gat 1017
Asn Trp Asp Gln Gly Trp Leu Ala Pro Gly Asp Trp Val Phe Lys Asp

305                 310                 315                 320

tct gtc agc tac gac tat gag cct cag aag gag tgg agt ccc tgg tct     1065
Ser Val Ser Tyr Asp Tyr Glu Pro Gln Lys Glu Trp Ser Pro Trp Ser
                325                 330                 335

ccc tgc agt ggg aac tgc agc act ggc aag cag cag agg act cgg ccc     1113
Pro Cys Ser Gly Asn Cys Ser Thr Gly Lys Gln Gln Arg Thr Arg Pro
            340                 345                 350

tgt ggc tat ggc tgc act gcc acc gag acc cgt acc tgt gac ctg ccc     1161
Cys Gly Tyr Gly Cys Thr Ala Thr Glu Thr Arg Thr Cys Asp Leu Pro
        355                 360                 365

tcc tgt cct ggc act gag gac aag gac acc ttg ggc ctc ccc agt gag     1209
Ser Cys Pro Gly Thr Glu Asp Lys Asp Thr Leu Gly Leu Pro Ser Glu
    370                 375                 380

gag tgg aag ctc ctg gcc cgc aat gct acg gac atg cat gat caa gat     1257
Glu Trp Lys Leu Leu Ala Arg Asn Ala Thr Asp Met His Asp Gln Asp
385                 390                 395                 400

gtg gac agc tgt gag aag tgg ctg aac tgc aag agc gac ttc cta atc     1305
Val Asp Ser Cys Glu Lys Trp Leu Asn Cys Lys Ser Asp Phe Leu Ile
                405                 410                 415

aag tat ctg agc cag atg ctg cgg gac ctg ccc agc tgc ccg tgt gcc     1353
Lys Tyr Leu Ser Gln Met Leu Arg Asp Leu Pro Ser Cys Pro Cys Ala
            420                 425                 430

tac cca ctg gag gcc atg gac agc cct gtg agc cta cag gac gag cac     1401
Tyr Pro Leu Glu Ala Met Asp Ser Pro Val Ser Leu Gln Asp Glu His
        435                 440                 445

cag ggc cgc agc ttc cgg tgg agg gat gcc agt ggc cct cgc gag cgc     1449
Gln Gly Arg Ser Phe Arg Trp Arg Asp Ala Ser Gly Pro Arg Glu Arg
    450                 455                 460

ctg gac atc tac cag ccc acg gcg cgc ttc tgc ctg cgt tcc atg ctg 1497
Leu Asp Ile Tyr Gln Pro Thr Ala Arg Phe Cys Leu Arg Ser Met Leu
465                 470                 475                 480

tct ggg gag agc agc aca ctg gcc gcc cag cac tgc tgc tat gac gag 1545
Ser Gly Glu Ser Ser Thr Leu Ala Ala Gln His Cys Cys Tyr Asp Glu
                485                 490                 495

gac agc cgg ctg ctg acc cgt ggc aag ggc gcc ggc atg ccc aac ctc 1593
Asp Ser Arg Leu Leu Thr Arg Gly Lys Gly Ala Gly Met Pro Asn Leu
            500                 505                 510

atc agc acc gac ttc tca cct aag ctg cac ttc aag ttc gac acg acg 1641
Ile Ser Thr Asp Phe Ser Pro Lys Leu His Phe Lys Phe Asp Thr Thr
        515                 520                 525

ccc tgg atc ctg tgc aag ggg gac tgg agc cgc ctc cac gct gtg ctc 1689
Pro Trp Ile Leu Cys Lys Gly Asp Trp Ser Arg Leu His Ala Val Leu
    530                 535                 540

cct ccc aac aac ggc cga gcc tgc acc gac aac ccc ctg gag gag gag 1737
Pro Pro Asn Asn Gly Arg Ala Cys Thr Asp Asn Pro Leu Glu Glu Glu
545                 550                 555                 560

tac cta gca cag ttg cag gag gcc aag gag tac tagtgacggg gttgctgaac 1790
Tyr Leu Ala Gln Leu Gln Glu Ala Lys Glu Tyr
                565                 570

agacactgca gggagagggc aggcggctgc tgctgttgca cgggagaact ttcctggtag 1850

ggccctcacc cgcccctgcc cagacagggt gaggaaaggg ctcccccagt gaggttggtc 1910

cgaggctgtg tgccctctgc cagcgacccc gaagcagata tctcagtggg gttagtgaga 1970

aggttgaagg gtatgtaggg cccagggtgg gtgtccctgg gagccctgga aatgtgcata 2030

tgtgcatgtg tctgccgggg cctccctctg ctgcctgctg ggaccctggc cactcatttt 2090

tctcctcctt gggagctggg ctcttctgcc ctggctctgc acataagtgt tagccagcag 2150

ctccagaaaa atcccgattc ccgggatctg ccacgagtca ctcctactcc accctgatgg 2210

ccagcagagg aagggccact cttctcatgg gcacagccat cctttgccgg gggggcatcc 2270

agcccgggtg gccacccctc cttatctctg ggtggtgcac atgcccttct ttccccactc 2330

cctgccacga gccactgcac aggaggctat ctgtagcccc aagctgcctt tctgttggac 2390

accaacttta gtcttgggct gcaagccagc ccagctgagg cgaagtggac tccaggcagg 2450

gaatgggttg cccaattctg gtccctttcc tttgctcagc cccctctgtt ctgctgattg 2510

tagggatgtg cagggctggg agttggcact ccccccgagt ggggaggtga cagcttgtca 2570

cagtagccag gcttgggtgg gttcagcact agctcgggac ggtgtgtcac acgtctatag 2630

taaaccagtt ctctgggagg ggaaaaaagc cctgatttat tgcatttggg cagcttctgt 2690

ggtgtaaatt ctcccagcag tgtcccatgt catgctgcca gcatcactga atgcactgaa 2750

ctcagagttg ggaagagatg cacataatcg ctctcccggc acacctcatg cctcttccct 2810

gcctccccat tcccctggct gcacttcctt gccttctatg gggttgaaat gttgaagtct 2870

caactgtctc tgttcacaag agccaccaaa agttagggga cttcagtcct agcccccaga 2930

tggccgccct gaagctctct gggctcctca gcaataaagc actttatttt c 2981

<210> 2
<211> 571
<212> PRT
<213> Homo sapiens

<400> 2
Met Arg Ala Leu Arg Asp Arg Ala Gly Leu Leu Leu Cys Val Leu Leu
1               5                   10                  15

Leu Ala Ala Leu Leu Glu Ala Ala Leu Gly Leu Pro Val Lys Lys Pro
            20                  25                  30

Arg Leu Arg Gly Pro Arg Pro Gly Ser Leu Thr Arg Leu Ala Glu Val
        35                  40                  45

Ser Ala Ser Pro Asp Pro Arg Pro Leu Lys Glu Glu Glu Glu Ala Pro
    50                  55                  60

Leu Leu Pro Arg Thr His Leu Gln Ala Glu Pro His Gln His Gly Cys
65                  70                  75                  80

Trp Thr Val Thr Glu Pro Ala Ala Met Thr Pro Gly Asn Thr Thr Pro
                85                  90                  95

Pro Arg Thr Pro Glu Val Thr Pro Leu Arg Leu Glu Leu Gln Lys Leu
            100                 105                 110

Pro Gly Leu Ala Ser Thr Thr Leu Ser Thr Pro Asn Pro Asp Thr Gln
        115                 120                 125

Ala Ser Ala Ser Pro Asp Pro Arg Pro Leu Arg Glu Glu Glu Glu Ala
    130                 135                 140

Arg Leu Leu Pro Arg Thr His Leu Gln Ala Glu Leu His Gln His Gly
145                 150                 155                 160

Cys Trp Thr Val Thr Glu Pro Ala Ala Leu Thr Pro Gly Asn Ala Thr
                165                 170                 175

Pro Pro Arg Thr Gln Glu Val Thr Pro Leu Leu Leu Glu Leu Gln Lys
            180                 185                 190

Leu Pro Glu Leu Val His Ala Thr Leu Ser Thr Pro Asn Pro Asp Asn
        195                 200                 205

Gln Val Thr Ile Lys Val Val Glu Asp Pro Gln Ala Glu Val Ser Ile
    210                 215                 220

Asp Leu Leu Ala Glu Pro Ser Asn Pro Pro Pro Gln Asp Thr Leu Ser
225                 230                 235                 240

Trp Leu Pro Ala Leu Trp Pro Phe Leu Trp Gly Asp Tyr Lys Gly Glu
                245                 250                 255

Glu Lys Asp Arg Ala Pro Gly Glu Lys Gly Glu Glu Lys Glu Glu Asp
            260                 265                 270

Glu Asp Tyr Pro Ser Glu Asp Ile Glu Gly Glu Asp Gln Glu Asp Lys
        275                 280                 285

Glu Glu Asp Glu Glu Glu Gln Ala Leu Trp Phe Asn Gly Thr Thr Asp
    290                 295                 300

Asn Trp Asp Gln Gly Trp Leu Ala Pro Gly Asp Trp Val Phe Lys Asp
305                 310                 315                 320

Ser Val Ser Tyr Asp Tyr Glu Pro Gln Lys Glu Trp Ser Pro Trp Ser
325. 330 335

Pro Cys Ser Gly Asn Cys Ser Thr Gly Lys Gln Gln Arg Thr Arg Pro
340 345 350

Cys Gly Tyr Gly Cys Thr Ala Thr Glu Thr Arg Thr Cys Asp Leu Pro
355 360 365

Ser Cys Pro Gly Thr Glu Asp Lys Asp Thr Leu Gly Leu Pro Ser Glu
370 375 380

Glu Trp Lys Leu Leu Ala Arg Asn Ala Thr Asp Met His Asp Gln Asp
385                 390                 395                 400

Val Asp Ser Cys Glu Lys Trp Leu Asn Cys Lys Ser Asp Phe Leu Ile
                405                 410                 415

Lys Tyr Leu Ser Gln Met Leu Arg Asp Leu Pro Ser Cys Pro Cys Ala
            420                 425                 430

Tyr Pro Leu Glu Ala Met Asp Ser Pro Val Ser Leu Gln Asp Glu His
        435                 440                 445

Gln Gly Arg Ser Phe Arg Trp Arg Asp Ala Ser Gly Pro Arg Glu Arg
    450                 455                 460

Leu Asp Ile Tyr Gln Pro Thr Ala Arg Phe Cys Leu Arg Ser Met Leu
465                 470                 475                 480

Ser Gly Glu Ser Ser Thr Leu Ala Ala Gln His Cys Cys Tyr Asp Glu
                485                 490                 495

Asp Ser Arg Leu Leu Thr Arg Gly Lys Gly Ala Gly Met Pro Asn Leu
            500                 505                 510

Ile Ser Thr Asp Phe Ser Pro Lys Leu His Phe Lys Phe Asp Thr Thr
        515                 520                 525

Pro Trp Ile Leu Cys Lys Gly Asp Trp Ser Arg Leu His Ala Val Leu
    530                 535                 540

Pro Pro Asn Asn Gly Arg Ala Cys Thr Asp Asn Pro Leu Glu Glu Glu
545                 550                 555                 560

Tyr Leu Ala Gln Leu Gln Glu Ala Lys Glu Tyr
                565                 570

<210> 3
<211> 30
<212> RNA
<213> Artificial Sequence

<220>
<223> Description of Artificial Sequence:Artificially
      Synthesized Oligo-cap Linker

<400> 3
agcaucgagu cggccuuguu ggccuacugg                                        30


<210> 4
<211> 42
<212> DNA
<213> Artificial Sequence

<220>
<223> Description of Artificial Sequence:Artificially
      Synthesized Primer Sequence

<400> 4
gcggctgaag acggcctatg tggccttttt tttttttttt tt                          42


<210> 5
<211> 21
<212> DNA
<213> Artificial Sequence

<220>
<223> Description of Artificial Sequence:Artificially
      Synthesized Primer Sequence

<400> 5
agcatcgagt cggccttgtt g                                                 21

<210> 6
<211> 21
<212> DNA
<213> Artificial Sequence

<220>
<223> Description of Artificial Sequence:Artificially
      Synthesized Primer Sequence

<400> 6
gcggctgaag acggcctatg t                                                21

<210> 7
<211> 27
<212> DNA
<213> Artificial Sequence

<220>
<223> Description of Artificial Sequence:Artificially
      Synthesized Primer Sequence

<400> 7
gcttctgcct gcgttccatg ctgtctg                                          27

<210> 8
<211> 27
<212> DNA
<213> Artificial Sequence

<220>
<223> Description of Artificial Sequence:Artificially
      Synthesized Primer Sequence

<400> 8
ggcacacagc ctcggaccaa cctcact                                          27

<210> 9
<211> 24
<212> DNA
<213> Artificial Sequence

<220>
<223> Description of Artificial Sequence:Artificially
      Synthesized Primer Sequence

<400> 9
ctccccgtga agaagccgcg gctc                                             24

<210> 10
<211> 32
<212> DNA
<213> Artificial Sequence

<220>
<223> Description of Artificial Sequence:Artificially
      Synthesized Primer Sequence

<400> 10
gcaagcttct agtactcctt ggcctcctgc aa                                    32